# EUROPÄISCHE PATENTSCHRIFT

(11) **EP 1 034 291 B1**
(45) Veröffentlichungstag und Bekanntmachung des Hinweises auf die Patenterteilung: **02.03.2005**
(21) Anmeldenummer: 98965602.0
(22) Anmeldetag: 27.11.1998
(51) Int. Cl.: C12N 15/86, A61K 48/00, C12N 5/10

(54) **RETROVIRALE PSEUDOTYP-VEKTOREN MIT MODIFIZIERTEN OBERFLÄCHEN-HÜLLPROTEINEN UND VERFAHREN ZU IHRER HERSTELLUNG FÜR DEN SELEKTIVEN GENTRANSFER**
PSEUDO-TYPE RETROVIRAL VECTORS WITH MODIFIED SURFACE CAPSID PROTEINS AND METHOD FOR THE PRODUCTION THEREOF FOR SELECTIVE GENE TRANSFER
VECTEURS RETROVIRAUX PSEUDOTYPES A PROTEINES DE SURFACE D'ENVELOPPE MODIFIEES ET PROCEDE DE FABRICATION DESDITS VECTEURS POUR LE TRANSFERT SELECTIF DE GENES

(30) Priorität: 28.11.1997 DE 19752855
(43) Veröffentlichungstag der Anmeldung: 13.09.2000
(73) Patentinhaber: Bundesrepublik Deutschland, letztvertreten durch den Präsidenten des Paul-Ehrlich-Instituts Prof. Dr. R. Kurth, 63225 Langen (DE)
(72) Erfinder: CICHUTEK, Klaus, D-60598 Frankfurt am Main (DE); MERGET-MILLITZER, Heike, D-65300 Seligenstadt (DE)
(74) Vertreter: Grund, Martin, Dr.
(86) Internationale Anmeldenummer: PCT/DE1998/003542
(87) Internationale Veröffentlichungsnummer: WO 1999/028488

(56) Entgegenhaltungen:
- WO-A-95/23846
- WO-A-96/30504
- VALSESIA-WITTMANN S ET AL: "IMPROVEMENT OF RETROVIRAL RETARGETING BY USING AMINO ACID SPACERS BETWEEN AN ADDITIONAL BINDING DOMAIN AND THE N TERMINUS OF MOLONEY MURINE LEUKEMIA VIRUS SU" JOURNAL OF VIROLOGY, Bd. 70, Nr. 3, 1. März 1996, Seiten 2059-2064, XP002037488
- CHU T-H T & DORNBURG R: "Towars highly efficientcell-type-specific gene transfer with retroviral vectors displaying single-chain antibodies" JOURNAL OF VIROLOGY, Bd. 71, Nr. 1, Januar 1997, Seiten 720-725, XP002104908 AMERICAN SOCIETY FOR MICROBIOLOGY US in der Anmeldung erwähnt
- SCHNIERLE BS ET AL: "Pseudotyping of murine leukemia virus with the envelope glycoproteins of HIV generates a retroviral vector with specificity of infection for CD4-expressing cells" PROCEEDINGS OF THE NATIONAL ACADEMY OF SCIENCES OF USA, Bd. 94, August 1997, Seiten 8640-8645, XP002104909 WASHINGTON US in der Anmeldung erwähnt
- SOMIA N V ET AL: "GENERATION OF TARGETED RETROVIRAL VECTORS BY USING SINGLE-CHAIN VARIABLE FRAGMENT: AN APPROACH TO IN VIVO GENE DELIVERY" PROCEEDINGS OF THE NATIONAL ACADEMY OF SCIENCES OF USA, Bd. 92, 1. August 1995, Seiten 7570-7574, XP000672863

### SEQUENZPROTOKOLL

(1) ALLGEMEINE ANGABEN:
   (i) ANMELDER:
      (A) NAME: Bundesrepublik Deutschland letztvertreten durch den Präsidenten des Paul-Ehrlich-Institute
      (B) STRASSE: Paul-Ehrlich-Str. 51-59
      (C) ORT: Langen
      (E) LAND: Deutschland
      (F) POSTLEITZAHL: 63225
   (ii) BEZEICHNUNG DER ERFINDUNG: Retrovirale Pseudotyp-Vektoren mit modifizierten Oberflächen-Hüllproteinen und Verfahren zu ihrer Herstellung für den selektiven Gentransfer
   (iii) ANZAHL DER SEQUENZEN: 32
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
   (v) DATEN DER FRUEHEREN ANMELDUNG:
      ANMELDENUMMER: DE 197 52 855.4
      ANMELDETAG: 28-11-1997
(2) ANGABEN ZU SEQ ID NO: 1:
   (i) SEQUENZKENNZEICHEN:
      (A) LÄNGE: 4776 Basenpaare
      (B) ART: Nucleotid
      (C) STRANGFORM: Doppelstrang
      (D) TOPOLOGIE: linear
   (ii) ART DES MOLEKÜLS: Sonstige Nucleinsäure
   (iii) HYPOTHETISCH: NEIN
   (xi) SEQUENZBESCHREIBUNG: SEQ ID NO: 1:
   GAATTCCCGT ACGAGCCATA GATAAAATAA AAGATTTTAT TTAGTCTCCA GAAAAAGGGG 60
   GGAATGAAAG ACCCCACCTG TAGGTTTGGC AAGCTAGCTT AAGTAACGCC ATTTTGCAAG 120
   GCATGGAAAA ATACATAACT GAGAATAGAG AAGTTCAGAT CAAGGTCAGG AACAGATGGA 180
   ACAGCTGAAT ATGGGCCAAA CAGGATATCT GTGGTAAGCA GTTCCTGCCC CGGCTCAGGG 240
   CCAAGAACAG ATGGAACAGC TGAATATGGG CCAAACAGGA TATCTGTGGT AAGCAGTTCC 300
   TGCCCCGGCT CAGGGCCAAG AACAGATGGT CCCCAGATGC GGTCCAGCCC TCAGCAGTTT 360
   CTAGAGAACC ATCAGATGTT TCCAGGGTGC CCCAAGGACC TGAAATGACC CTGTGCCTTA 420
   TTTGAACTAA CCAATCAGTT CGCTTCTCGC TTCTGTTCGC GCGCTTCTGC TCCCCGAGCT 480
   CAATAAAAGA GCCCACAACC CCTCACTCGG GGCGCCAGTC CTCCGATTGA CTGAGTCGCC 540
   CGGGTGGGGG AGCTCGCTGT TGGGCTCGCG GTTGAGGACA AACTCTTCGC GGTCTTTCCA 600
   GTACTCTTGG ATCGGAAACC CGTCGGCCTC CGAACGGTAC TCCGCCACCG AGGGACCTGA 660
   GCGAGTCCGC ATCGACCGGA TCGGAAAACC TCTCGAGAAA GGCGTCTAAC CAGTCACAGT 720
   CGCAAGGTAG GCTGAGCACC GTGGCCGGGC GGCACGGGTG GCGGTCGGGG TTGTTTCTGG 780
   CGGAGGTGCT GCTGATGATG TAATTAAGTA GGCGGTCTTG AGACGGCGAT GGTCGAGGTG 840
   AGGTGTGGCA GGCTTGAGAT CTGGCCATAC ACTTGAGTGA CAATGACATC CACTTTGCCT 900
   TTCTCTCCAC AGGTGTCCAC TCCCAGGTCC AACCGGATCC GAGCTCCACC GCGGTAAAGG 960
   TCGCTGGGAA GACCCCGTGG ATCCACCACT CTCGACTCAA GAAAGCTCCT GACAACCAAG 1020
   AAGAATGGAC TGTCTCACCA ACCTCCGATC CGCTGAGGGT AAAGTTGACC AGGCGAGCAA 1080
   AATCCTAATT CTCCTTGTGG CTTGGTGGGG GTTTGGGACC ACTGCCGAAG TTTCGACTGC 1140
   CGGCTCCGGG GGCGGTGGTT CTGGTGGTGG TTCTGGTGGT GGTGGTTCTG GTGGTGGTGG 1200
   TTCTGGCGCC AGCCCAGTCC AGTTTATCCC CCTGCTTGTG GGTCTAGGGA TTTCAGGGGC 1260
   TACACTTGCT GGTGGAACGG GGCTTGGGGT CTCCGTTCAC ACTTATCACA AGCTCTCTAA 1320
   TCAATTGATT GAAGATGTCC AGGCTCTTTC AGGGACCATC AATGACCTAC AGGACCAGAT 1380
   TGACTCCCTG GCTGAGGTTG TCTTACAAAA TAGAAGAGGG TTAGACCTAT TGACTGCCGA 1440
   ACAAGGAGGA ATATGTCTCG CACTCCAGGA GAAGTGTTGT TTTTACGCTA ACAAGTCGGG 1500
   TATCGTACGT GACAAGATCC GAAAACTCCA AGAGGACCTT ATCGAGAGAA AACGTGCACT 1560
   GTACGACAAC CCCCTGTGGA GCGGCTTGAA CGGCTTCCTT CCATATTTGC TACCCTTGTT 1620
   AGGCCCCCTG TTTGGGCTCA TATTGTTCCT GACCCTCGGC CCGTGCATTA TGAAGACCCT 1680
   GACTCGCATT ATACATGACA AAATTCAGGC AGTAAAATCC TAGCACTAGT CCCACAGTAC 1740
   AAGCCACTCC CAACAGAGAT GGATACCCTA GGGGTCCGAT GGTCTAAGAA TTCTCGAGTC 1800
   TAAGATCGAT CGAATTCCTA GGTCAATGAT TTGACCAGAA TGTACAAGAG CAGTGGGGAA 1860
   TGTGGGAGGG GCTTACGAAG GCCTTAAGTG ACTAGGTACC CGATCCAGAC ATGATAAGAT 1920
   ACATTGATGA GTTTGGACAA ACCACAACTA GAATGCAGTG AAAAAAATGC TTTATTTGTG 1980
   AAATTTGTGA TGCTATTGCT TTATTTGTAA CCATTATAAG CTGCAATAAA CAAGTTAACA 2040
   ACAACAATTG CATTCATTTT ATGTTTCAGG TTCAGGGGGA GGTGTGGGAG GTTTTTTAAA 2100
   GCAAGTAAAA CCTCTACAAA TCAAGCTGGG CAAGCTAGAT CTAGCTTGGC GTAATCATGG 2160
   TCATAGCTGT TTCCTGTGTG AAATTGTTAT CCGCTCACAA TTCCACACAA CATACGAGCC 2220
   GGAAGCATAA AGTGTAAAGC CTGGGGTGCC TAATGAGTGA GCTAACTCAC ATTAATTGCG 2280
   TTGCGCTCAC TGCCCGCTTT CCAGTCGGGA AACCTGTCGT GCCAGCTGCA TTAATGAATC 2340
   GGCCAACGCG CGGGGAGAGG CGGTTTGCGT ATTGGGCGCT CTTCCGCTTC CTCGCTCACT 2400
   GACTCGCTGC GCTCGGTCGT TCGGCTGCGG CGAGCGGTAT CAGCTCACTC AAAGGCGGTA 2460
   ATACGGTTAT CCACAGAATC AGGGGATAAC GCAGGAAAGA ACATGTGAGC AAAAGGCCAG 2520
   CAAAAGGCCA GGAACCGTAA AAAGGCCGCG TTGCTGGCGT TTTTCCATAG GCTCCGCCCC 2580
   CCTGACGAGC ATCACAAAAA TCGACGCTCA AGTCAGAGGT GGCGAAACCC GACAGGACTA 2640
   TAAAGATACC AGGCGTTTCC CCCTGGAAGC TCCCTCGTGC GCTCTCCTGT TCCGACCCTG 2700
   CCGCTTACCG GATACCTGTC CGCCTTTCTC CCTTCGGGAA GCGTGGCGCT TTCTCAATGC 2760
   TCACGCTGTA GGTATCTCAG TTCGGTGTAG GTCGTTCGCT CCAAGCTGGG CTGTGTGCAC 2820
   GAACCCCCCG TTCAGCCCGA CCGCTGCGCC TTATCCGGTA ACTATCGTCT TGAGTCCAAC 2880
   CCGGTAAGAC ACGACTTATC GCCACTGGCA GCAGCCACTG GTAACAGGAT TAGCAGAGCG 2940
   AGGTATGTAG GCGGTGCTAC AGAGTTCTTG AAGTGGTGGC CTAACTACGG CTACACTAGA 3000
   AGGACAGTAT TTGGTATCTG CGCTCTGCTG AAGCCAGTTA CCTTCGGAAA AAGAGTTGGT 3060
   AGCTCTTGAT CCGGCAAACA AACCACCGCT GGTAGCGGTG GTTTTTTTGT TTGCAAGCAG 3120
   CAGATTACGC GCAGAAAAAA AGGATCTCAA GAAGATCCTT TGATCTTTTC TACGGGGTCT 3180
   GACGCTCAGT GGAACGAAAA CTCACGTTAA GGGATTTTGG TCATGAGATT ATCAAAAAGG 3240
   ATCTTCACCT AGATCCTTTT AAATTAAAAA TGAAGTTTTA AATCAATCTA AAGTATATAT 3300
   GAGTAAACTT GGTCTGACAG TTACCAATGC TTAATCAGTG AGGCACCTAT CTCAGCGATC 3360
   TGTCTATTTC GTTCATCCAT AGTTGCCTGA CTCCCCGTCG TGTAGATAAC TACGATACGG 3420
   GAGGGCTTAC CATCTGGCCC CAGTGCTGCA ATGATACCGC GAGACCCACG CTCACCGGCT 3480
   CCAGATTTAT CAGCAATAAA CCAGCCAGCC GGAAGGGCCG AGCGCAGAAG TGGTCCTGCA 3540
   ACTTTATCCG CCTCCATCCA GTCTATTAAT TGTTGCCGGG AAGCTAGAGT AAGTAGTTCG 3600
   CCAGTTAATA GTTTGCGCAA CGTTGTTGCC ATTGCTACAG GCATCGTGGT GTCACGCTCG 3660
   TCGTTTGGTA TGGCTTCATT CAGCTCCGGT TCCCAACGAT CAAGGCGAGT TACATGATCC 3720
   CCCATGTTGT GCAAAAAAGC GGTTAGCTCC TTCGGTCCTC CGATCGTTGT CAGAAGTAAG 3780
   TTGGCCGCAG TGTTATCACT CATGGTTATG GCAGCACTGC ATAATTCTCT TACTGTCATG 3840
   CCATCCGTAA GATGCTTTTC TGTGACTGGT GAGTACTCAA CCAAGTCATT CTGAGAATAG 3900
   TGTATGCGGC GACCGAGTTG CTCTTGCCCG GCGTCAATAC GGGATAATAC CGCGCCACAT 3960
   AGCAGAACTT TAAAAGTGCT CATCATTGGA AAACGTTCTT CGGGGCGAAA ACTCTCAAGG 4020
   ATCTTACCGC TGTTGAGATC CAGTTCGATG TAACCCACTC GTGCACCCAA CTGATCTTCA   4080
   GCATCTTTTA CTTTCACCAG CGTTTCTGGG TGAGCAAAAA CAGGAAGGCA AAATGCCGCA   4140
   AAAAAGGGAA TAAGGGCGAC ACGGAAATGT TGAATACTCA TACTCTTCCT TTTTCAATAT   4200
   TATTGAAGCA TTTATCAGGG TTATTGTCTC ATGAGCGGAT ACATATTTGA ATGTATTTAG   4260
   AAAAATAAAC AAATAGGGGT TCCGCGCACA TTTCCCCGAA AAGTGCCACC TGACGTCTAA   4320
   GAAACCATTA TTATCATGAC ATTAACCTAT AAAAATAGGC GTATCACGAG GCCCTTTCGT   4380
   CTCGCGCGTT TCGGTGATGA CGGTGAAAAC CTCTGACACA TGCAGCTCCC GGAGACGGTC   4440
   ACAGCTTGTC TGTAAGCGGA TGCCGGGAGC AGACAAGCCC GTCAGGGCGC GTCAGCGGGT   4500
   GTTGGCGGGT GTCGGGGCTG GCTTAACTAT GCGGCATCAG AGCAGATTGT ACTGAGAGTG   4560
   CACCATATGC GGTGTGAAAT ACCGCACAGA TGCGTAAGGA GAAAATACCG CATCAGGCGC   4620
   CATTCGCCAT TCAGGCTGCG CAACTGTTGG GAAGGGCGAT CGGTGCGGGC CTCTTCGCTA   4680
   TTACGCCAGC TGGCGAAAGG GGGATGTGCT GCAAGGCGAT TAAGTTGGGT AACGCCAGGG   4740
   TTTTCCCAGT CACGACGTTG TAAAACGACG GCCAGT                             4776
(2) ANGABEN ZU SEQ ID NO: 2:
   (i) SEQUENZKENNZEICHEN:
      (A) LÄNGE: 12 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 2:
   Met Lys Asp Pro Thr Cys Arg Phe Gly Lys Leu Ala
                   5                   10
(2) ANGABEN ZU SEQ ID NO: 3:
   (i) SEQUENZKENNZEICHEN:
      (A) LÄNGE: 21 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 3:
   Met Glu Lys Tyr Ile Thr Glu Asn Arg Glu Val Gln Ile Lys Val Arg
   5 10 15
   Asn Arg Trp Asn Ser
   20
(2) ANGABEN ZU SEQ ID NO: 4:
   (i) SEQUENZKENNZEICHEN:
      (A) LÄNGE: 8 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 4:
   Met Gly Gln Thr Gly Tyr Leu Trp
   5
(2) ANGABEN ZU SEQ ID NO: 5:
   (i) SEQUENZKENNZEICHEN:
      (A) LÄNGE: 13 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 5:
   Met Glu Gln Leu Asn Met Gly Gln Thr Gly Tyr Leu Trp
   5 10
(2) ANGABEN ZU SEQ ID NO: 6:
   (i) SEQUENZKENNZEICHEN:
      (A) LÄNGE: 12 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 6:
   Met Val Pro Arg Cys Gly Pro Ala Leu Ser Ser Phe
   5 10
(2) ANGABEN ZU SEQ ID NO: 7:
   (i) SEQUENZKENNZEICHEN:
      (A) LÄNGE: 10 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 7:
   Met Phe Pro Gly Cys Pro Lys Asp Leu Lys
                   5                   10
(2) ANGABEN ZU SEQ ID NO: 8:
   (i) SEQUENZKENNZEICHEN:
      (A) LÄNGE: 15 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 8:
   Met Val Glu Val Arg Cys Gly Arg Leu Glu Ile Trp Pro Tyr Thr
                   5                   10                  15
(2) ANGABEN ZU SEQ ID NO: 9:
   (i) SEQUENZKENNZEICHEN:
      (A) LÄNGE: 24 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 9:
   Met Thr Ser Thr Leu Pro Phe Ser Pro Gln Val Ser Thr Pro Arg Ser
                   5                   10                  15
   Asn Arg Ile Arg Ala Pro Pro Arg
               20
(2) ANGABEN ZU SEQ ID NO: 10:
   (i) SEQUENZKENNZEICHEN:
      (A) LÄNGE: 232 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 10:
   Met Asp Cys Leu Thr Asn Leu Arg Ser Ala Glu Gly Lys Val Asp Gln
   5 10 15
   Ala Ser Lys Ile Leu Ile Leu Leu Val Ala Trp Trp Gly Phe Gly Thr
   20 25 30
   Thr Ala Glu Val Ser Thr Ala Gly Ser Gly Gly Gly Gly Ser Gly Gly
   35 40 45
   Gly Ser Gly Gly Gly Gly Ser Gly Gly Gly Gly Ser Gly Ala Ser Pro
   50 55 60
   Val Gln Phe Ile Pro Leu Leu Val Gly Leu Gly Ile Ser Gly Ala Thr
   65 70 75 80
   Leu Ala Gly Gly Thr Gly Leu Gly Val Ser Val His Thr Tyr His Lys
   85 90 95
   Leu Ser Asn Gln Leu Ile Glu Asp Val Gln Ala Leu Ser Gly Thr Ile
   100 105 110
   Asn Asp Leu Gln Asp Gln Ile Asp Ser Leu Ala Glu Val Val Leu Gln
   115 120 125
   Asn Arg Arg Gly Leu Asp Leu Leu Thr Ala Glu Gln Gly Gly Ile Cys
   130 135 140
   Leu Ala Leu Gln Glu Lys Cys Cys Phe Tyr Ala Asn Lys Ser Gly Ile
   145 150 155 160
   Val Arg Asp Lys Ile Arg Lys Leu Gln Glu Asp Leu Ile Glu Arg Lys
   165 170 175
   Arg Ala Leu Tyr Asp Asn Pro Leu Trp Ser Gly Leu Asn Gly Phe Leu
   180 185 190
   Pro Tyr Leu Leu Pro Leu Leu Gly Pro Leu Phe Gly Leu Ile Leu Phe
   195 200 205
   Leu Thr Leu Gly Pro Cys Ile Met Lys Thr Leu Thr Arg Ile Ile His
   210 215 220
   Asp Lys Ile Gln Ala Val Lys Ser
   225 230
(2) ANGABEN ZU SEQ ID NO: 11:
   (i) SEQUENZKENNZEICHEN:
      (A) LÄNGE: 14 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 11:
   Met Asp Thr Leu Gly Val Arg Trp Ser Lys Asn Ser Arg Val
   5 10
(2) ANGABEN ZU SEQ ID NO: 12:
   (i) SEQUENZKENNZEICHEN:
      (A) LÄNGE: 15 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 12:
   Met Tyr Lys Ser Ser Gly Glu Cys Gly Arg Gly Leu Arg Arg Pro
   5 10 15
(2) ANGABEN ZU SEQ ID NO: 13:
   (i) SEQUENZKENNZEICHEN:
      (A) LÄNGE: 16 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 13:
   Met Ile Arg Tyr Ile Asp Glu Phe Gly Gln Thr Thr Thr Arg Met Gln
   5 10 15
(2) ANGABEN ZU SEQ ID NO:14:
   (i) SEQUENZKENNZEICHEN:
      (A) LÄNGE: 4 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 14:
   Met Leu Tyr Leu

(2) ANGABEN ZU SEQ ID NO: 15:
   (i) SEQUENZKENNZEICHEN:
      (A) LÄNGE: 6 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 15:
   Met Leu Leu Leu Tyr Leu
   5
(2) ANGABEN ZU SEQ ID NO: 16:
   (i) SEQUENZKENNZEICHEN:
      (A) LÄNGE: 12 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 16:
   Met Phe Gln Val Gln Gly Glu Val Trp Glu Val Phe
   5 10
(2) ANGABEN ZU SEQ ID NO: 17:
   (i) SEQUENZKENNZEICHEN:
      (A) LÄNGE: 26 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 17:
   Met Val Ile Ala Val Ser Cys Val Lys Leu Leu Ser Ala His Asn Ser
   5 10 15
   Thr Gln His Thr Ser Arg Lys His Lys Val
   20 25
(2) ANGABEN ZU SEQ ID NO: 18:
   (i) SEQUENZKENNZEICHEN:
      (A) LÄNGE: 49 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 18:
   Met Ser Glu Leu Thr His Ile Asn Cys Val Ala Leu Thr Ala Arg Phe
   5 10 15
   Pro Val Gly Lys Pro Val Val Pro Ala Ala Leu Met Asn Arg Pro Thr
   20 25 30
   Arg Gly Glu Arg Arg Phe Ala Tyr Trp Ala Leu Phe Arg Phe Leu Ala
   35 40 45
   His
(2) ANGABEN ZU SEQ ID NO: 19:
   (i) SEQUENZKENNZEICHEN:
      (A) LÄNGE: 4 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 19:
   Met Leu Thr Leu
(2) ANGABEN ZU SEQ ID NO: 20:
   (i) SEQUENZKENNZEICHEN:
      (A) LÄNGE: 9 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 20:
   Met Arg Leu Ser Lys Arg Ile Phe Thr
   5
(2) ANGABEN ZU SEQ ID NO: 21:
   (i) SEQUENZKENNZEICHEN:
      (A) LÄNGE: 11 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 22:
(2) ANGABEN ZU SEQ ID NO: 23:
   (i) SEQUENZKENNZEICHEN:
      (A) LÄNGE: 88 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 23:
   Met Ile Pro Arg Asp Pro Arg Ser Pro Ala Pro Asp Leu Ser Ala Ile
                   5                   10                  15
   Asn Gln Pro Ala Gly Arg Ala Glu Arg Arg Ser Gly Pro Ala Thr Leu
               20                  25                  30
   Ser Ala Ser Ile Gln Ser Ile Asn Cys Cys Arg Glu Ala Arg Val Ser
           35                  40                  45
   Ser Ser Pro Val Asn Ser Leu Arg Asn Val Val Ala Ile Ala Thr Gly
       50                  55                  60
   Ile Val Val Ser Arg Ser Ser Phe Gly Met Ala Ser Phe Ser Ser Gly
   65                  70                  75                  80
   Ser Gln Arg Ser Arg Arg Val Thr
                   85
(2) ANGABEN ZU SEQ ID NO: 24:
   (i) SEQUENZKENNZEICHEN:
      (A) LÄNGE: 56 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 24:
   Met Leu Cys Lys Lys Ala Val Ser Ser Phe Gly Pro Pro Ile Val Val
                   5                   10                  15
   Arg Ser Lys Leu Ala Ala Val Leu Ser Leu Met Val Met Ala Ala Leu
               20                  25                  30
   His Asn Ser Leu Thr Val Met Pro Ser Val Arg Cys Phe Ser Val Thr
           35                  40                  45
   Gly Glu Tyr Ser Thr Lys Ser Phe
       50                  55
(2) ANGABEN ZU SEQ ID NO: 25:
   (i) SEQUENZKENNZEICHEN:
      (A) LÄNGE: 49 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 25:
   Met Arg Arg Pro Ser Cys Ser Cys Pro Ala Ser Ile Arg Asp Asn Thr
                   5                   10                  15
   Ala Pro His Ser Arg Thr Leu Lys Val Leu Ile Ile Gly Lys Arg Ser
               20                  25                  30
   Ser Gly Arg Lys Leu Ser Arg Ile Leu Pro Leu Leu Arg Ser Ser Ser
           35                  40                  45
   Met
(2) ANGABEN ZU SEQ ID NO: 26:
   (i) SEQUENZKENNZEICHEN:
      (A) LÄNGE: 6 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 26:
   Met Pro Gln Lys Arg Glu
                   5
(2) ANGABEN ZU SEQ ID NO: 27:
   (i) SEQUENZKENNZEICHEN:
      (A) LÄNGE: 27 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 27:
   Met Leu Asn Thr His Thr Leu Pro Phe Ser Ile Leu Leu Lys His Leu
                   5                   10                  15
   Ser Gly Leu Leu Ser His Glu Arg Ile His Ile
               20                  25
(2) ANGABEN ZU SEQ ID NO: 28:
   (i) SEQUENZKENNZEICHEN:
      (A) LÄNGE: 5 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 28:
   Met Tyr Leu Glu Lys
                     5
(2) ANGABEN ZU SEQ ID NO: 29:
   (i) SEQUENZKENNZEICHEN:
      (A) LÄNGE: 26 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 29:
   Met Thr Leu Thr Tyr Lys Asn Arg Arg Ile Thr Arg Pro Phe Arg Leu
                     5                  10                  15
   Ala Arg Phe Gly Asp Asp Gly Glu Asn Leu
                20                  25
(2) ANGABEN ZU SEQ ID NO: 30:
   (i) SEQUENZKENNZEICHEN:
      (A) LÄNGE: 11 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 30:
   Met Gln Leu Pro Glu Thr Val Thr Ala Cys Leu
                     5                  10
(2) ANGABEN ZU SEQ ID NO: 31:
   (i) SEQUENZKENNZEICHEN:
      (A) LÄNGE: 31 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 31:
   Met Pro Gly Ala Asp Lys Pro Val Arg Ala Arg Gln Arg Val Leu Ala
   5 10 15
   Gly Val Gly Ala Gly Leu Thr Met Arg His Gln Ser Arg Leu Tyr
   20 25 30
(2) ANGABEN ZU SEQ ID NO:32:
   (i) SEQUENZKENNZEICHEN:
      (A) LÄNGE: 65 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 32:

## Beschreibung

Die Erfindung betrifft retrovirale Pseudotyp-Vektoren mit modifizierten Oberflächen-Hüllproteinen, die für die zellspezifische Transduktion eines ausgewählten Säugerzelltyps geeignet sind (Zelltargeting), Verfahren zur Herstellung der zellspezifischen retroviralen Pseudotyp-Vektoren und ihre Verwendung zur Genübertragung in ausgewählte Zellen.

Ziel der somatischen Gentherapie soll der effektive Transfer von Genen oder Gen-Fragmenten sein, die funktionelle Homologie zu einem defekten Gen aufweisen, oder von Genen oder Gen-Fragmenten mit therapeutischer Wirkung. Bisherige Versuche und klinische Studien zur somatischen Gentherapie wurden überwiegend auf Basis der retroviralen Murinen Leukämieviren (MLV) durchgeführt. Der Wirtszellbereich retroviraler Vektoren wird durch das Oberflächen-Hüllprotein (SU) bestimmt, das vom *env*-Gen codiert wird. Die Proteinprodukte des *env*-Gens bilden die äußere Hülle des retroviralen Vektors. Die SU-Proteine interagieren mit, d.h. sie binden an ein bestimmtes Protein (Rezeptor) auf der Oberfläche der Wirtszelle. Die *env*-Genprodukte z.B. des amphotropen MLV erlauben den Gentransfer in eine große Anzahl unterschiedlicher Säugerzellen. Sowohl mittels ecotroper als auch amphotroper MLV-Vektoren werden generell alle murinen (ecotrop) bzw. murine und humane Zellen (amphotrop) transduziert, da die von diesen Viren angesteuerten Rezeptoren ubiquitär vorhanden sind. Ein zellspezifischer Gentransfer mittels MLV ist somit nicht möglich.

Eine Wirtszellspezifität ist z.B. für den gentherapeutischen Einsatz jedoch von Vorteil, da bei einer Gentherapie außerhalb des Organismus (*ex vivo*) (Anderson et al., *Science* 256 (1992), 808-813; Yu et al., *H*. *Gene Therapy* 8 (1997),1065-1072) aufwendige Aufreinigungen von Zellen vermieden werden. Für den Therapie-, Diagnostik- oder Impf-Einsatz *in vivo* ist erwünscht, daß die retroviralen Vektoren gezielt die gewünschten Wirtszellen ansteuern, die von genetischen Fehlfunktionen betroffen bzw. Ziel der Therapie sind, und anschließend das therapeutische Gen übertragen.

Eine Einengung des Wirtszellbereichs z.B. des amphotropen MLV konnte durch Modifikation des Oberflächenhüllproteins erreicht werden. Eine Modikation des Oberflächenhüllproteins wurde durch die Fusion mit einer Hormondomäne durchgeführt. Es fand eine Transduktion der Zellen statt, die den spezifischen Hormonrezeptor trugen (Kasahara *et al*., *Science* 266 (1994), 1373-1375). Ferner wurde das Oberflächenhüllprotein durch Fusion mit einem einkettigen Antikörperfragment (*single chain variable fragment*, nachfolgend auch "scFv" bezeichnet) modifiziert. Das Fragment repräsentierte die antigenbindende Domäne eines Antikörpers und ist ein Fusionsprotein, das aus den variablen Domänen V_{H} und V_{L} eines monoklonalen Antikörpers zusammengesetzt ist. Die beiden Domänen sind über ein Glycin- und Serin-Oligopeptid [-(ser-gly4)3-gly-)] verknüpft, das die korrekte Faltung des Fusionsproteins ermöglicht (Huston et al., *Methods Enzymol*. 203 (1991), 46-88; Whitlow et al., *Methods: A companion to Methods Enzymol*. 2 (1991), 97-105). Alle bisher durchgeführten Modifikationen des MLV-Oberflächenhüllproteins mit einem scFv zeigten, daß es zwar zu einer Bindung der Vektoren an die Wirtszielzelle kam, nicht jedoch zu einem Eintritt in die Zelle (Russel et al., *Nucleic Acid Res.* 21 (1993), 1081-1985). Weiterhin ist bekannt, daß das Oberflächenhüllprotein des MLV generell keine umfangreichen Modifikationen erlaubt (Cosset et al., *J*. *Virol* 69 (1995), 6314-632). Modifikationen, bei denen ein Teil der Bindungsdomäne des MLV-SU-Proteins ersetzt wurde, führten oft zu einer inkorrekten Prozessierung und somit zu einem defekten Transport des SU-Proteins an die Zelloberfläche (Weiss et al., *In J*.*A*. *Levy* (ed.), *The Retroviridae* 2 (1993), 1-108 ; Morgan et al., *J*. *Virol*. 67 (1993), 4712-4721; Russel et al., *Nucleic Acid Res.* 21 (1993), 1081-1085).

Um die Problematik der nötigen Modifikation des Oberflächenhüllproteins, die zur spezifischen Ansteuerung des gewünschten Zelltyps nötig ist, zu umgehen, wurde die Herstellung wirtszellspezifischer retroviraler Vektoren durch die Pseudotypsierung von z. B. MLV-Kapsiden erreicht. Dabei stammt der Viruskem vom MLV und die Virushülle (SU-Proteine) von anderen retroviralen Vektoren. Vorteile der Pseudotypsierung von MLV-Kapsiden liegen in der Minderung des Risikos der Entstehung von replikationskompetenten Retroviren. Durch Verwendung unterschiedlicher nicht homologer Expressionsplasmide, die für die Strukturgene *gag*, *pol* und *env* kodieren wird die Gefahr der Rekombination vermindert. So konnte gezeigt werden, daß MLV-Kapside mit ENV-Proteinen des Affensarkom assoziierten Virus (Takeuschi et al., *Virology* 186 (1992), 792-794), des Katzen Leukämie Virus (Porter et al., *Hum*. *Gene Ther*. 7 (1996), 913-919) bzw. der endogenen Retroviren der Katzen (Vile et al., *Virology* 180 (1991), 420-424) pseudotypisiert werden können. Reiser et al. (*Proc*. *Natl*. *Acad. Sci*. *USA* 93 (1996), 15266-15271) konnten zeigen, daß die Herstellung von HIV/MLV bzw. HIV/VSV-G Pseudotypen möglich ist. Ebenso wurde die mögliche Inkorporation von VSV-G-Protein (Bums et al., *Proc*. *Natl*. *Acad*. *Sci*. *USA* 90 (1993), 8033-8037; Ory et al., *Proc*. *Natl*. *Acad*. *Sci*. *USA* 93 (1996), 11400-11406,) sowie die Inkorporation von trunkierten HIV-env-Glykoproteinen (Schnierle et al., *Proc. Natl*. *Acad*. *Sci*. *USA* 94 (1997), 8640-8645) in MLV-Kapside beschrieben. Die in dieser Weise pseudotypisierten Vektoren können jedoch nicht jeden belieben zellspezifischen Rezeptor eines Zelltyps anzusteuern, da die Zielzelle nur durch den Tropismus (Zellspezifität eines Virus) des jeweiligen Virus-Oberflächenproteins bestimmt wird.

Der Gentransfer in Säugerzellen mittels (pseudotypisierten Retroviren) retroviraler Vektoren hat generell folgende Vorteile:
- Es wird in der Regel eine Kopie des gewünschten Gens in die Säugerzelle überführt.
- Das gewünschte Gen wird im allgemeinen ohne Mutation oder Rearrangements übertragen.
- Es erfolgt ein stabiler Einbau des gewünschten Gens in das Genom der Zielzelle.

Der Gentransfer in Säugerzellen mittels pseudotypisierten Retroviren hat weiterhin den Vorteil, daß mittels Pseudotypsisierung von z.B. MLV-, HIV-, Foamyvirus- oder SIV-Kapsiden vor allem mit SNV-ENV-Protein eine gezielte Veränderung der Zellspezifität der jeweiligen retroviralen Vektoren vorgenommen werden kann, so daß z.B. ein therapeutisches Gen in eine ausgewählte Zellpolpulation eingeführt werden kann.

Der Erfindung liegt daher die Aufgabe zugrunde, retrovirale Pseudotyp-Vektoren mit modifizierten Oberflächen-Hüllproteinen, die für die zellspezifische Transduktion eines ausgewählten Säugerzelltyps geeignet sind (Zelltargeting) bereitzustellen. Der Erfindung liegt ferner die Aufgabe zugrunde, ein Verfahren zur Herstellung der zellspezifischen retroviralen Pseudotyp-Vektoren bereitzustellen. Der Erfindung liegt ferner die Aufgabe zugrunde, retrovirale Verpackungszellen zur Gewinnung der erfindungsgemäßen Vektoren bereitzustellen. Mit Hilfe dieser Vektoren gelingt es, im Gegensatz zu den bisher bekannten Vektoren, jeden beliebigen Rezeptor einer Zielzelle anzusteuern.

Die Aufgabe der Erfindung wird durch die erfindungsgemäßen retrovirale Vektoren, enthaltend einen Viruskern von murinen Leukämievirus (MLV) und eine Virushülle vom Milz-Nekrosevirus (SNV) gelöst. Bevorzugt sind retrovirale Vektoren, deren Virushüllen das Vollängen-Oberflächenprotein (SU-Protein) des SNV und/oder ein chimäres SNV-virusfremdes Polypeptid-ENV, SNV-HIV-ENV oder SNV-SIV-ENV umfassen. Besonders bevorzugt sind retrovirale Vektoren, deren virusfremdes Polypeptid ein Ligand, ein Peptidfragment eines Liganden, ein Antikörper, ein Peptidfragment eines Antikörpers oder eine Antikörpererkennungsdomäne (scFv) umfaßt. Ferner bevorzugt sind retrovirale Vektoren, weiter umfassend eine RNA, die in die durch den retroviralen Vektor zu transduzierende Zelle eingeführt werden soll. Besonders bevorzugt sind retrovirale Vektoren, deren RNA ein therapeutisches Gen oder ein Nukleinsäurefragment eines therapeutischen Gens und/oder ein Reportergen umfaßt. Insbesondere bevorzugt sind retrovirale Vektoren, wobei das therapeutische Gen oder das Nukleinsäurefragment eines therapeutischen Gens das CFTR-Gen, phox91, ADA, IL-16, p53, transdominante Mutanten (z.B. revM10) sowie Impfgene z. B. rekombinantes gp120 und IL-16 umfaßt. Weiter besonders bevorzugt sind retrovirale Vektoren, wobei das Reportergen β-Galaktosidase, "Green Fluorescent Protein", Luciferase oder die Resistenzgene Neomycin oder "multiple drug resistence gene" umfaßt. Die erfindungsgemäßen retroviralen Vektoren können als Arzneimittel verwendet werden. Bevorzugt ist die Verwendung zur Herstellung eines Arzneimittels zur somatischen Gentherapie, Impftherapie oder Diagnostik. Besonders bevorzugt ist die Therapie der Cystischen Fibrose, des ADA-Mangels, der chronischen Granulomatose und der HIV-1 Infektion.

Die Aufgabe der Erfindung wird ferner durch die erfindungsgemäßen retroviralen Verpackungszellen zur Gewinnung der erfindungsgemäßen retroviralen Vektoren gelöst. Die erfindungsgemäßen retroviralen Verpackungszellen sind sowohl mit einem oder mehreren psi-negativen Expressionskonstrukt(en), die die gag- und pol-Genprodukte des MLV, exprimieren als auch mit einem psi-negativen SNV-Env- und/oder psi-negativen SNV-Env-virusfremden Polypeptid-, psi-negativen SNV-HIV-Env- oder SNV-SIV-env-Expressionskonstrukt transfiziert. Bevorzugt ist eine retrovirale Verpackungszelle, in der das virusfremde Polypeptid des psi-negativen SNV-Env-virusfremden Polypeptid-Expressionskonstrukts einen Ligand, ein Peptidfragment eines Liganden, einen Antikörper, ein Peptidfragment eines Antikörpers oder eine Antikörpererkennungsdomäne (scFv) umfaßt. Ferner bevorzugt ist eine retrovirale Verpackungszellinie, ferner umfassend ein psi-positives Expressionskonstrukt, umfassend eine Nukleinsäuresequenz, die in die durch den retroviralen Vektor zu transduzierende Zelle eingeführt werden soll. Besonders bevorzugt ist eine retrovirale Verpackungszellinie, wobei die Nukleinsäuresequenz ein therapeutisches Gen oder dessen Nukleinsäurefragment und/oder ein Reportergen umfaßt. Insbesondere bevorzugt ist eine retrovirale Verpackungszellinie, wobei das therapeutische Gen oder das NukJeinsäurefragment eines therapeutischen Gens das CFTR-Gen, phox91, ADA, IL-16, p53, transdominante Mutanten (z.B. revM10) und Impfgene z. B. rekombinantes gp120 und IL-16 umfaßt. Ferner besonders bevorzugt ist eine retrovirale Verpackungszellinie, wobei das Reportergen β-Galaktosidase, "Green Flourescent Protein", Luciferase oder die Resistenzgene Neomycin oder das "multiple drug resistence gene" umfaßt.

### Die Abbildungen dienen der Erläuterung der Erfindung

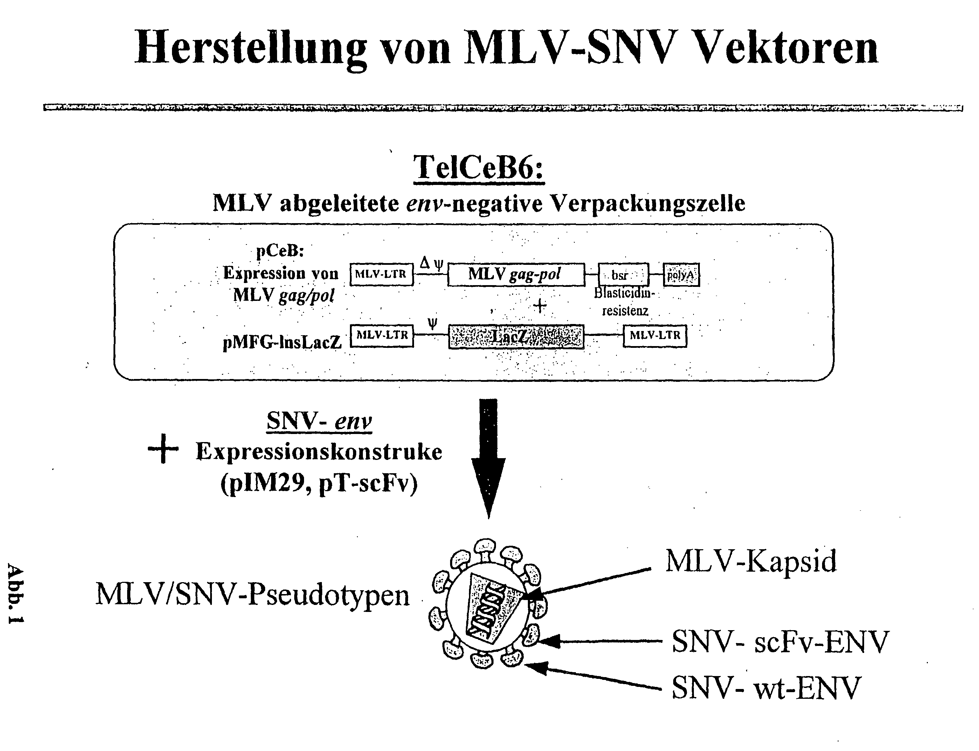

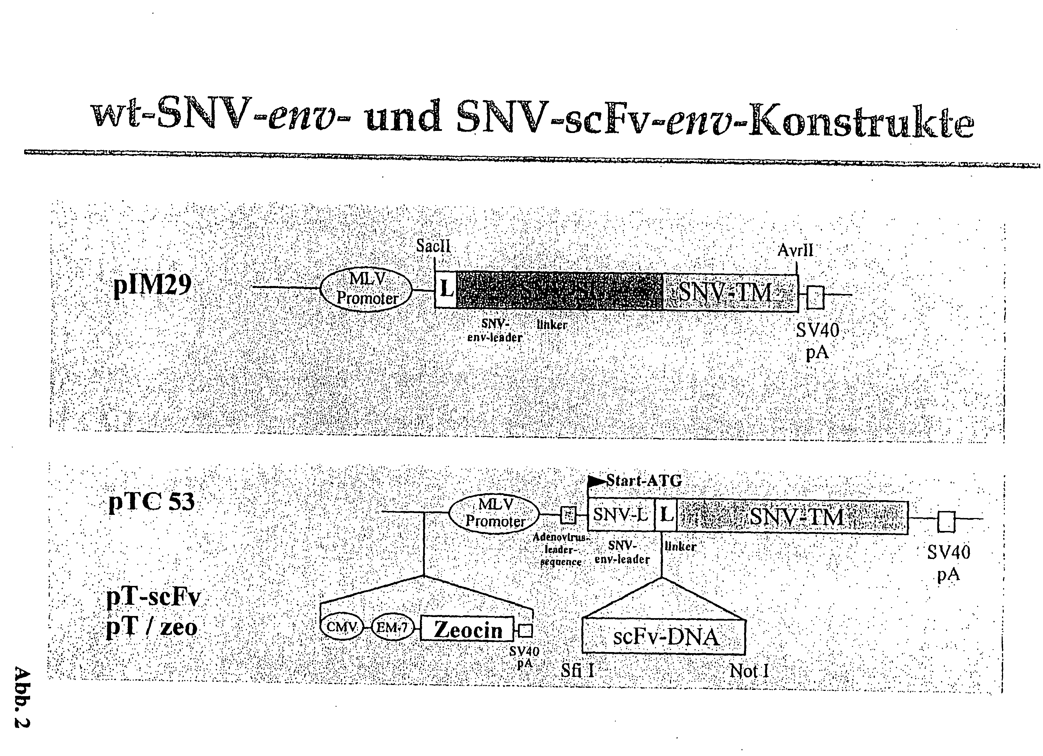

Abb.1 zeigt schematisch die Gewinnung eines MLV/SNV-Pseudotyp-Vektors. Die Verpackungszelle TelCeB6 enthält die Konstrukte pCeB und pMFG-InsLacZ. Die Zelle exprimiert somit die Strukturgene *gag* und *pol ,* sowie das Reportergen *β-Galaktosidase*. Zur Herstellung eines pseudotysierten Vektors werden die SNV-env-Expressionskonstrukte (siehe Abb. 2) in die Zelle transfiziert. Hiermit werden ebenfalls die Sturkturgene für das wt-*env* (pTM29; Chu et al., 1997) und die chimären scFv-*env* (pT-scFv) zur Verfügung gestellt und exprimiert. Es werden Vektorpartikel in den Zellkulturüberstand abgegeben, die aus MLV-Kapsiden bestehen und SNV-env-Proteine in die Virushülle inkorporieren. Im MLV-Kapsid ist nur die pMFG-lnsLacZ-RNA verpackt, so daß das Genprodukt dieses Reportergens nach erfolgreichem Gentransfer (Transduktion der Zielzellen) nachgewiesen werden kann.
Abb 2 zeigt schematisch das SNV-wt-*env*-Gen kodierende Konstrukt pIM29 als auch die chimären SNV-scFv- *env*-Gen Konstrukte.

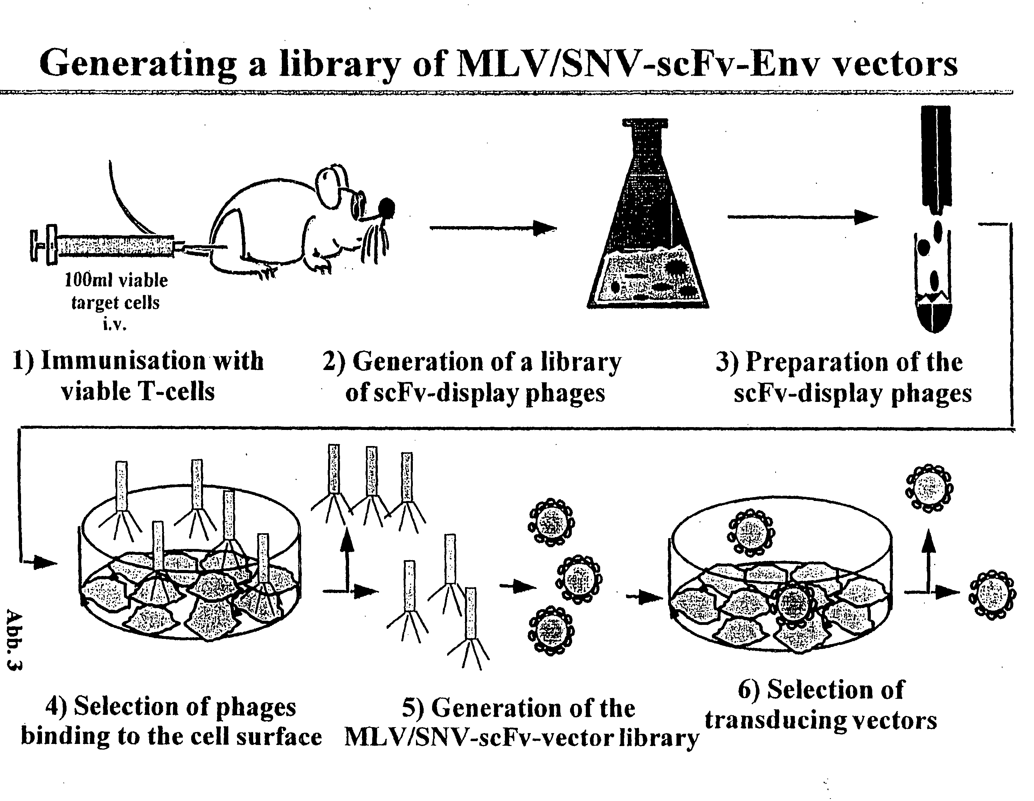

Abb.3 zeigt schematisch die Herstellung, Isolierung und Selektion der erfindungsgemäßen Vektoren.
Abb. 4 zeigt die Nukleinsäuresequenz von pTC53.

Der hier verwendete Begriff amphotropes Virus bedeutet Infektion und Replikation auf murinen und humanen Zellen, im Gegensatz zu ecotropen Viren, das nur auf murinen Zellen repliziert. Der hier verwendete Begriff retroviraler Vektor bedeutet replikationsdefizientes retrovirales Viruspartikel, das anstelle der retroviralen mRNA eine fremde eingeführte RNA eines Gens, z.B. eines therapeutischen Gens oder dessen Fragment oder eines Reportergens übertragen kann. Der hier verwendete Begriff pseudotypisiert bedeutet, daß der retrovirale Vektor einen Viruskern eines Retrovirus besitzt und die Virushülle von einem anderen Retrovirus stammt. Der hier verwendete Begriff Antikörpererkennungsdomäne (scFv) bedeutet Antigenbindestelle eines Antikörpers, umfassend Vh- und Vl-Kette.

Zur Bereitstellung der erfindungsgemäßen pseudotypisierten retroviralen Vektoren wird zunächst ein Expressinsprodukt hergestellt, welche die GAG- und POL-Genprodukte des MLV umfaßt. Das Expressionskonstrukt ist psi-negativ, d.h. das Verpackungssignal psi (ψ) ist deletiert. Somit wird das für die Gene gag und pol kodierende Expressionskonstrukt z.B. pCeB (Cosset et al., *J. Virol* 69 (1995), 6314-632), nicht in die entstehenden retroviralen Vektoren verpackt, so daß ein nicht replikationskompetentes Virus entsteht. Als weitere Komponente dient das *env*-Gen des SNV. Durch Modifikation des Oberflächen-Hüllproteins (SU-Proteins) z. B. durch Insertion eines Antikörpers, Antikörperfragments, scFv's oder eines Liganden eines Oberflächen-Rezeptors von z.B. humanen Zellen oder anderer virusfremder Polypeptide, kann der ursprüngliche Tropismus von SNV (aviane Zellen) verändert werden. Im Gegensatz zu dem MLV-Oberflächenprotein, das nur mäßige Modifikation (Weiss et al., *In J*.*A*. *Levy* (ed.), *The Retroviridae* 2 (1993), 1-108 ; Morgan et al., *J*. *Virol*. 67 (1993), 4712-4721; Russel et al., *Nucleic Acid Res.* 21 (1993), 1081-1085), kann das gesamte SNV-Oberflächenprotein durch ein virusfremdes Polypeptid ersetzt werden, ohne daß die Prozessierung z.B. eines chimären SNV- scFv-ENV-Proteins beeinträchtigt wird (Martinez und Dornburg, 1994; Chu und Dornburg, *J. Virol*. 71 (1997), 720-725). Die virusfremden Polypeptide, z.B. die scFv-Domänen, im Oberflächen-Hüllprotein vermitteln die Erkennung und die Anbindung an Oberflächenproteine ausgewählter Zellpopulationen, z.B. von hämatopoietischen Zellen, T-Zellen, Leberzellen, Epithelzellen, Muskelzellen oder Fibroblastenvon z.B. Mensch, Maus, Ratte, Schaf oder Rind. Zur effizienten Transduktion der ausgewählten Zellen können neben den modifizierten ENV-Proteinen auch die Wildtyp-SNV-ENV-Proteine in die MLV-Kapside inkorporiert werden. Auch das für das Wildtyp-SNV-ENV-Protein kodierende Plasmid muß psi-negativ sein, damit entsprechende Boten-RNA nicht in die Retroviruspartikel aufgenommen wird. Die erfindungsgemäßen HIV/SNV- bzw. Foamyvirus/SNV-Pseudotypen sind z. B. zum spezifischen Gentransfer in ruhende ausdifferenzierte Zellen geeignet (Naldini et al., *Science* 272 (1996), 263-267; Lindemann et al., *J*. *Virol*. 71 (1997), 48715-4820).

Ein weiteres Expressionsprodukt enthält eine DNA-Sequenz des Genprodukts, das in die durch den pseudotypisierten retroviralen Vektor zu transduzierende Zelle eingeführt werden soll. Die DNA-Sequenz kann ein therapeutisches Gen, ein Gen-Fragment eines therapeutischen Gens, ein DNA-Fragment eines in der Zielzelle mutierten Gens oder ein Markergen sein. Typische Beispiele für eine DNA-Sequenz sind das CFTR-Gen, das ADA-Gen, das LDL-Rezeptor-Gen, β-Globin-Gen, Faktor-VIII-Gen oder Faktor-IX-Gen oder das Dystrophin-Gen. Im Falle des CFTR-Gens wären die Zielzellen des erfindungsgemäßen Vektors z.B. die Lungenepithelzellen, beim ADA-Gen die Stammzellen des Knochenmarks oder T-Lymphocyten, beim LDL-Rezeptor die Leberzellen, beim Dystrophin-Gen die Skelettmuskelzellen, beim β-Globin-Gen die hämatopoietischen Stammzellen, beim Faktor VIII oder Faktor IX die und Leberzellen. Dem Fachmann ist ersichtlich, daß diese Aufzählung nur eine Auswahl der therapeutischen Gene darstellt und andere Gene ebenfalls in die erfindungsgemäße Verpackungszelle zur Verpackung in die erfindungsgemäßen Vektoren transfiziert werden können. Die DNA-Fragmente eines therapeutischen Gens umfassen z.B. Antisense-Nukleinsäuren oder Ribozyme. DNA-Fragmente eines in der Zielzelle mutierten Gens können ferner Bereiche eines Gens umfassen, die die Trinukleotidwiederholungen von z.B. des Fragile X Gens umfassen. Ein Markergen oder Reportergen ist, z.B. β-Galaktosidase, "Green Flourescent Protein", Luciferase oder das Resistenzgen Neomycin. Die Herstellung solcher Expressionskonstrukte ist Stand der Technik, z.B. enthält das vom MLV abstammende Expressionskonstrukt MFG-InsLacZ die cDNA des β-Galaktosidase-Reportergens Reportergens (Takeuchi et al. *J*. *Virol.* 68 (1994), 8001-8007). Um den Gentransfer zu erreichen, muß die Verpackung dieses Expressionsplasmids in den retroviralen Vektor gewährleistet sein. Charakteristisch für ein solches Expressionskonstrukt ist daher das Vorhandensein einer Verpackungsstelle (psi, ψ).

Die Konstruktion der gag- und pol-Expressionsprodukte für MLV, HIV, SIV, Foamyvirus ist Stand der Technik (Naldini et a., *Science* 272 (1996), 263-267; Ory, D.S., *Proc. Natl*. *Acad*. *Sci*. *USA* 93 (1996), 11400-11406; Poeschla et al., *Proc*. *Natl*. *Acad Sci*. *USA* 93 (1996), 11395-11399; Buchschacher et al., *J. Virol*. 66 (1992), 2731-2739; Poznansky et al., 1991; Mammano et al., *J*. *Virol*. 71 (1997), 3341-3345).

Die Konstruktion der für die wt-SNV-ENV-Proteine z.B. pIM29 und die chimären SNV-scFv-ENV-Proteine kodierenden Expressionplasmide ist von Chu et al. (*J*. *Virol*. 71 (1997), 720-725) vorbeschrieben. Die Expression der für das wt-env-Gen kodierenden DNA wird von einem MLV-Promotor gesteuert. Die env-cDNA wurde über die Restriktionsschnittstellen SacII und AvrII aus einem für das komplette SNV-Virus kodierenden Plasmids ausgeschnitten und durch Insertion in einen Linker (L) eingefügt. Um eine korrekte Prozessierung des Proteins zu gewährleisten, enthält pIM29 die Polyadenylierungsstelle des Simianen Virus 40 (SV40). Von diesem Plasmid kann somit die Expression des wt-env-Gens erfolgen, so daß nach proteolytischer Spaltung eines Vorläuferproteins das äußere Glykoprotein (SU) und das Transmembranprotein (TM) vorliegt. Es können jedoch andere, dem Fachmann bekannte Plasmide, Promotoren, Linker, Polyadenylierungssignale und weitere für eine korrekte Prozessierung benötigte DNA-Elemente verwendet werden.

Zur Exprimierung von SNV-scFv-ENV-Proteinen werden die in bekannter Weise erhaltenen scFv in ein SNV-ENV-Expressionskonstrukt z.B. pTC53 in üblicher Weise eingeführt. Die in pTC53 vorhandenen Restriktionserkennungstellen für die Enzyme SfiI und NotI ermöglichen die molekulare Klonierung von z.B. scFv's zwischen SNV-env-Leader-Sequenz und dem für das Transmembranprotein (TM-Protein) kodierenden Bereich der DNA. Die im wt-ENV vorhandene Proteaseschnittstelle zwischen SU und TM ist in pTC53 deletiert, so daß ein Fusionsprotein exprimiert wird, das N-terminal aus dem einkettigen Antikörperframgent und C-terminal aus dem SNV-TM besteht.

Die regulatorischen Elemente wie MLV-Promotor und SV40-Polyadenylierungssignal sind identisch mit denen des pIM29-Vektors. Zur Verstärkung der Expression eines chimären env-Gens wird in dem Expressionsplasmid pTC53 eine adenovirale Leader-Sequenz z.B. AVtl (Sheay et al. *BioTechniques*, 15 (1993), 856-861) inseriert. Eine Zoezin-Kasette (pSV2zeo; Fa. Invitrogen, Niederlande) dient der möglichen Selektionierung von stabil transfizierten Zellen, so daß einzelne Zellklone etabliert werden können.

Beliebige Antikörpererkennungsdomänen, die spezifisch für jede gewünschte Zielzelle sind, können hergestellt werden, indem eine kombinatorische Phagen-cDNA-Bibliothek der variablen Domänen der leichten und schweren Ketten der Immunglobuline hergestellt wird. Dazu wird ein Säugetier, z.B. eine Maus, Ratte, ein Kaninchen, Meerschweinchen, Ziege oder Schaf, mit einem ausreichenden Titer einer Zellpopulation in üblicher Weise immunisiert. Die Zellpopulation ist die Zellart, die einen Oberflächenrezeptor ausbildet, an den die scFv spezifisch binden. Die Zellen können von einem von dem zu immunisierenden Säugetier verschiedenen Säugetier stammen, z.B. vom Menschen, der Maus, Ratte, dem Schaf, Rind oder Schwein. Die Zellen können solche Zellen sein, in der z.B. eine Gentherapie, eine Impftherapie oder Diagnostik durchgeführt werden soll. Für die Immunisierung kann eine Zellpopulation oder mehrere Zellpopulationen gleichzeitig dem Säugetier verabreicht werden, je nachdem für welche Zellpopulationen der scFv spezifisch sein soll.

Für die Herstellung der cDNA-Bibliothek wird zuerst die B-Zell-RNA des immunisierten Säugetiers in bekannter Weise isoliert. Die mRNA-Sequenzen der für die Antigenerkennung verantwortlichen Regionen der schweren und leichten Kette (V_{H} und V_{L}) der Immunglobuline werden mittels reverser Transkription und anschließender Polymerase-Kettenamplifikation in üblicher Weise in cDNA umgeschrieben und vervielfältigt. Die Primerpaare und deren Sequenzen für die V_{H}- und V_{L}-Regionen sind dem Fachmann bekannt Sie sind z.B. im kommerziell erhältlichen Kit der Fa. Pharmacia enthalten, bzw. können den bekannten Datenbanken (EMBL) entnommen werden. Dem Fachmann ist bekannt, daß er für jede immunisierte Säugetierart verschiedene Primersequenzen verwenden muß. Die Sequenzen sind ebenfalls in den bekannten Datenbanken enthalten. Die cDNA-Fragmente der V_{H}- und V_{L}-Regionen werden dann mittels einer Ligasereaktion in üblicher Weise zu scFv-cDNAs verknüpft. Für den Fachmann ist ersichtlich, daß bei der Ligation unterschiedliche Kombinationen von cDNA-Fragmenten hergestellt werden. Die erhaltenen scFv-cDNAs können dann in einen Phagemid-Vektor z.B. pCANTA 5E Phagemid, Fa. Pharmacia kJoniert werden. Anschließend werden Wirtsbakterien z.B. E.coli TGl mit dem Phagemid-Vektor transformiert.

Die von den Bakterien produzierten rekombinanten Phagen werden dann in üblicher Weise isoliert und auf das Vorhandensein von zellspezifischen scFv-Peptiden selektioniert. Die Phagen werden mit der Zellpopulation oder den Zellpopulationen in üblicher Weise in Kontakt gebracht, die für die Immunisierung verwendet worden sind. Die Phagen, die nicht an die Zellen binden, tragen kein spezifisches scFv-Peptid und werden durch Waschschritte in üblicher Weise entfernt. Die Phagen, die an die Zellen binden, präsentieren das gewünschte scFv-Peptid auf ihrer Oberfläche und werden in üblicher Weise eluiert. Die Pagen, die das gewünschte scFv-Peptid präsentieren, werden vermehrt, indem man sie wieder in üblicher Weise die Wirtsbakterien infizieren läßt. Dieser Selektionsschritt kann ein oder mehrere Male wiederholt werden, um die bindenden Phagen anzureichern. Dieser Vorgang wird als "panning" bezeichnet. Die Phagen werden nach dem panning oder direkt nach dem ersten Selektionsschritt einer weiteren Selektion unterzogen. Dabei werden die Phagen mit einer oder mehreren anderen Zellpopulationen in Kontakt gebracht, die sich von den zur Immunisierung verwendeten Zellen unterscheiden. Die Phagen, die nicht an diese Zellen binden, präsentieren ein zellspezifisches scFv-Peptid. Sie werden in üblicher Weise aus dem Zellüberstand isoliert und für eine Wirtsbakterieninfektion zur Vermehrung verwendet. Auch dieser Selektionsschritt kann ein oder mehrere Male wiederholt werden (Marks et al., *Biotechnologie* 10 (1992), 779; Clackson et al., *Nature* 352 (1991), 624; Marks et al., *J. Mol*. *Biol*. 222 (1991), 581; Chaudhary et al., *Proc*. *Natl*. *Acad*. *Sci*. *USA* 87 (1990), 1066; Chiswell et al., *TIBTECH* 10 (1992), 80; MeCafferty et al., *Nature* 348 (1990), 552; Huston et al., *Proc. Natl*. *Acad Sci*. *USA* 85 (1988), 5879).

Die auf die beschriebene Weise selektionierten Phagen bilden die Ausgangsmaterialien zur Herstellung einer Vektor-Genbank für die erfindungsgemäßen pseudotypisierten retroviralen Vektoren. Jeder Vektor des Typs [MLV/SNV-scFv-Env] enthält somit in seiner Hülle eine bestimmte scFv-Domäne. Aus der Vektor-Genbank wurden dann jene pseudotypisierten retroviralen Vektoren ausgesucht, welche den Gentransfer in die ausgewählten Zielzellen, d.h. die Zellen, mit dem das Säugetier immunisiert wurde, bewerkstelligen können. Dazu werden einzelne [MLV/SNV-scFv-Env]-Vektoren mit einem einzigen scFv in der Hülle oder Pools derartiger Vektoren hergestellt und in Bezug auf ihre Fähigkeit, Gene in die ausgewählten Zellen zu übertragen, untersucht. Nur jene Vektoren und damit die zur Herstellung dieser Vektoren benutzten scFv werden auf die beschriebene Weise ausgewählt, die den gezielten Gentransfer in die ausgewählten Zielzellen durchführen.

Ferner kann das SNV-Oberflächenprotein außer mit virusfremden Polypeptiden auch durch das externe Glykoprotein (SIVagm) des Simianen Immundefizienzvirus (SIV) der Afrikanischen Grünen Meerkatze (Cercopithecus aethiops) oder des HIV-1- oder HIV-2-ENV-Proteins ersetzt werden. Solche chimären SNV/SIV-ENV-Proteine bzw. SNV/HIV-ENV-Proteine können ebenfalls in die MLV-Kapside in der vorstehend beschriebenen Weise inkorporiert werden. Diese erfindungsgemäßen pseudotypisierten retroviralen Vektoren können zu einem zellspezifischen Gentransfer in CD4-positive Lymphozyten eingesetzt werden.

Die erfindungsgemäße retrovirale Verpackungszelle zur Gewinnung der erfindungsgemäßen pseudotypisierten retroviralen Vektoren wird bereitgestellt, indem eine Zellinie, z.B. eine humane Zellinie mit den vorstehend beschrieben psi-negativen Expressionskonstrukten, die für die gag- und pol-Genprodukte des MLV exprimieren, und mit dem psi-negativen SNV-Env-Expressionskonstrukt und/oder psi-negativen SNV-Env-virusfremden Polypeptid-Expressionskonstrukt, SNV/HIV- oder SNV/SIV-Expressionskonstrukt in üblicher Weise transfiziert wird.

Ferner können Verpackungszellen verwendet werden, die bereits die psi-negativen Expressionskonstrukte für die gag und pol-Genprodukte enthalten. Ein typisches Beispiel einer solchen MLV-abgeleiteten Verpackungszelle ist TelCeB6 (Cosset et al., *J*. *Virol* 69 (1995), 6314-632). In solche Verpackungszellen müssen dann nur das psi-negative Expressionskonstrukt für die Virushülle und das psi-positive Expressionskonstrukt für die in die Zielzelle zu transduzierende Nukleinsäuresequenz transfiziert werden. Dem Fachmann sind die Verfahren zur Transfektion der Expressionskonstrukte bekannt. Von den erfindungsgemäßen Verpackungszellen werden retrovirale Vektorpartikel in den Zellüberstand abgegeben, die das Expressionskonstrukt enthalten nicht jedoch die Konstrukte, die für die GAG-, POL- und ENV-Proteine kodieren. In die Zielzelle wird somit nur das gewünschte z.B. therapeutische Gen oder Reportergen überführt. Die gezielte Transduktion von ausgewählten Zielzellen z.B. mittels SNV/SNV-scFv-Env-Vektoren konnte bisher mit unterschiedlichen scFv gezeigt werden (Chu et al., *Gene Therapie* 1 (1994), 292-299; Chu et al., *BioTechniques* 18 (1995), 890-899). Für die Etablierung einer stabilen Verpackungszellinie wird ein Zeozinkonstrukt eingeführt und die Verpackungszellen in üblicher Weise selektioniert.

Die Etablierung von stabilen Verpackungszellinien, die sowohl das wt-SNV-ENV als auch das chimäre SNV-ENV, SNV/HIV-ENV oder SNV/SIV-ENV-Expressionskonstrukte stabil exprimieren, erlaubt die Generierung von hochtitrigen Vektorstocks. Diese sind für ein gezieltes Zelltargeting wünschenswert.

Die dargestellte Erfindung eröffnet die folgenden Möglichkeiten:
- Gene, Gen-Fragmente oder sonstige Nukleinsäuresequenzen können in ausgewählte Säugerzellen überführt werden,
- weitere Effizienzsteigerungen des Nukleinsäuretransfers durch Verbesserung der env-Genkonstnikte können erreicht werden,
- Gentherapie-, Markierungs- und Impfstrategien können entwickelt werden, für die ein selektiver Nukleinsäuretransfer in ausgewählte Säugerzellen wünschenswert ist.

Die folgenden Beispiele erläutern die Erfindung und sind nicht als einschränkend aufzufassen:

### 1. Isolierung und Klonierung zellspezifischer scFv

Zur Herstellung, Isolierung und Selektion von. zellspezifischen scFv wurde eine Maus mit der humanen T-Zellinie T-C8166 (Clapham et al., *Virology* 158 (1987), 44-51) in üblicher Weise immunisiert, die Milz entfernt und die RNA isoliert. Die Klonierung der scFv-cDNAs wurde mit dem kommerziell erhältlichen Kit der Fa. Pharmacia nach Angaben des Herstellers durchgeführt. Die erhaltenen Phagen wurden in üblicher Weise auf ihre Bindungseigenschaften gegenüber den Zielzellen untersucht.

### 2. Klonieren der spezifischen scFv-cDNA-Fragmente in Env-Expressionskonstrukte

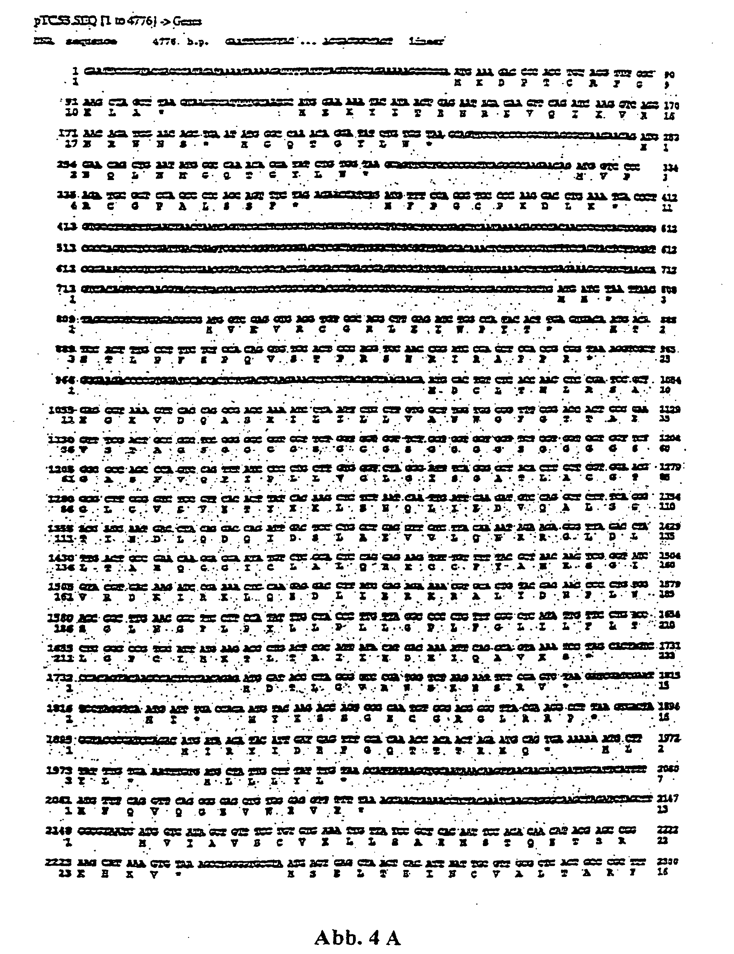

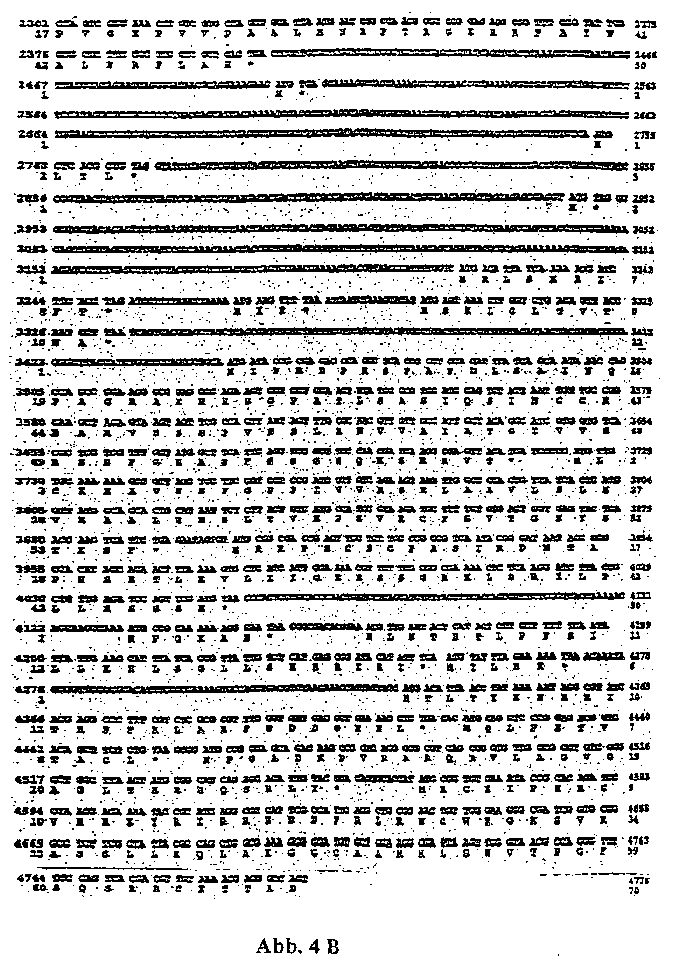

Vier so erhaltene zellspezifische scFv (M8, K6, 7A5, 7E10) wurden verwendet, um pseudotypisierte MLV-SNV-scFv-Vektoren herzustellen. Die scFv-cDNAs der vorstehend bezeichneten zellspezifischen scFv wurden in üblicher Weise aus der Phagemid-DNA ausgeschnitten und in das Expressionskonstrukt pTC53 ligiert. pTC53 wurde erhalten durch Modifzierung des universellen eukaryotischen Vektors pRD114 (Chu et al., *J. Virol*. 71 (1997), 720-725; Sheay et al. *BioTechniques*, 15 (1993), 856-861; Chu et al., *BioTechniques*, 18 (1995). 890-895). In diesem Vektor wurde das SNV-wt-env-Gen bis auf die für die Leader-Sequenz und das Transmembran-Protein kodierende cDNA deletiert. Ein zusätzlich eingeführter Spacer ermöglicht die Insertion einer Fremd-DNA (hier die scFv-cDNA) im Anschluß an die ENV-Leader-Sequenz über die Restriktionserkennungsstelle NaeI. Die Sequenz von pTC53 ist in Abbildung 4 gezeigt. Für die Insertion der scFv-cDNA wurde das Env-Expressionskonstrukt pTC53 dahingehend modifiziert, daß Sfi I und Not I spezifische Restriktionsendonuklease-Erkennungsstellen in den Linker zwischen der SNV-Leader-Sequenz und der SNV-Transmembran-Sequenz (TM) in üblicher Weise eingefügt werden. Hierzu wird eine rekombinante PCR ausgehend von der DNA des Plasmids PKA1558 (Scov H. & Andersen K.B., 1993) und der für das anti Transferrinrezeptor-scFv kodierenden DNA in üblicher Weise durchgeführt, so daß über Nru I (5'und 3') eine Insertion des amplifizierten Fragments in das Nae I restringierte pTC53 möglich ist. Das so inserierte Fragment enthält die multiple Sfi I / Not I Klonierungsstelle, da die verwendeten Primer neben der endständigen Nru I Erkennungsstelle weiterhin eine benachbarte Sfi I bzw. Not I Erkennungsstelle beinhalten. Für die rekombinante PCR wurden folgende Primer verwendet

Die PCR-Bedingungen waren: 94°C/3 min, 94°C/1 min, 59°C/1 min, 72°C/1 min., 25 X Schleifen, 72°C/10 min und dann bis 4°C abkühlen. Das PCR-Fragment wurde gelelektrophoretisch aufgetrennt, aus der Gelmatrix extrahiert (Quiaex, Fa. Quiagen) und mit dem Nae I geöffneten Plasmid pTC53 in üblicher Weise ligiert.

Die scFv-cDNAs aus dem Phagemid (*pCANTA 5E*) wurden mittels der Restriktionsendonukleasen Sfi I und Not I ausgeschnitten. Dazu wurde Phagemid-Plasmid-DNA mittels bekannter Verfahren hergestellt. und jeweils 8µg Plasmid-DNA mit jeweils 60 U der Restriktionsendonukleasen Sfi I und Not I für 1,5h bei 50°C und anschließend 1,5h bei 37°C verdaut. Der Reaktionsansatz fand in einem Volumen von 200µl statt, der mit 20µl BSA (10fach konz.) und 20µl Reaktionspuffer 3 (10fach konz.) supplementiert wurde. Nach Beendigung der Reaktionszeit wurde der Ansatz in einem 1%igem Agarosegel elektrophoretisch aufgetrennt. Nach der Auftrennung wurde die scFv-cDNA spezifische Bande (ca 750bp) aus dem Agarosegel mittels bekannter Verfahren aufgereinigt.

Das aufgereinigte Fragment wurde mit dem ebenfalls mit den Reastriktionsendonukleasen Sfi I und Not I geöffneten Env-Expressionskonstrukt pTC53 ligiert. Dazu wurden äquimolare Mengen des scFv-cDNA-Fragments und pTC53-Fragments in einem 15µl Volumen mit 200 U T4-Ligase und 1,5µl 10-fach Ligase-Puffer supplementiert. Der Ansatz wurde bei 4°C. Über Nacht inkubiert. Um eine effiziente Transformation von Bakterien zu ermöglichen, wurden die Bakterienstämme TOP10F' und JS5 mit einer nach Hanahan (1983) modifizierten Methode kompetent gemacht. Nach dem Animpfen von 100 ml LB-Medium mit 500 µl einer Übernachtkultur wurde die Bakteriensuspension bei 37° C bis zu einer Dichte (OD₅₅₀) von 0,6 inkubiert. Anschließend wurden die Bakterien auf Eis gekühlt, bei 6.000 rpm und 4° C pelletiert (Minifuge RF, Heraeus, Hanau) und in 40 ml TFB1-Puffer (30 mM KOAc, 100mM RbCl₂, 10 mM CaCl₂, 15 % Glycerin, pH 5,8 mit Essigsäure eingestellt, danach sterilfiltriert) resuspendiert. Nach einer Inkubationszeit von 15 Minuten auf Eis und einer Zentrifugation bei 6.000 rpm und 4° C wurde das Bakterienpellet in 4 ml TFB2-Puffer (10 mM MOPS, 75 mM CaCl₂, 10 mM RbCl₂, 15 % Glycerin, pH 6,5 mit KOH-Lösung eingestellt, danach sterilfiltriert)resuspendiert. Die Bakteriensuspension wurde dann in Aliquots je 100 µl aufgeteilt und auf Trockeneis schockgefroren. Die Lagerung erfolgte bei -70° C. Zur Transformation wurden 100 µl der kompetenten Bakterien auf Eis aufgetaut und nach Zugabe von 1-2 µl des jeweiligen Ligationsansatzes für 30 Minuten auf Eis inkubiert. Nach einem anschließenden Temperaturschock (45 s bei 42° C anschließend 2 min auf Eis) wurden die Bakterien mit 500 µl SOC-Medium (GIBCO/BRL, Eggenstein) vesetzt und bei 37° C für eine Stunde zur Expression der Antibiotikaresistenz in einem Bakterienschüttler kultiviert. Die Bakteriensuspension wurde auf LB-Agarplatten, die mit dem Antibiotikum Ampicillin supplementiert waren, ausgestrichen und bei 37° C über Nacht inkubiert.

Die Präparation von Plasmiden aus Bakterien (E.coli TopF10) erfolgte mit den QIAGEN-Plasmid-Kits der Firma QIAGEN, Hilden. Für die Präparation einer geringen Menge Plasmid-DNA wurden die Bakterien einer 15 ml-Übernachtkultur (LB-Medium mit 50 µg/ml Ampicillin) mit den vom Hersteller gelieferten Lösungen lysiert und über eine Anionenaustauscher-Säule (tip-20) gereinigt. Zur Gewinnung großer Mengen Plasmid-DNA (Maxi-Präparation) wurden 400 ml Übernachtkulturen angesetzt.

### 2. Transfektion der Verpackungszellen

Zunächst wurde getestet, ob eine Inkorporation des wt-SNV-ENV-Proteins in die MLV-Kapside erfolgt. Hierfür wurden 2µg DNA des Expressionskontruktes pIM29 (Dornburg, *Gene Therapie* 2 (1995), 1-10), das für das wt-ENV-Protein kodiert, durch eine liposomenvermittelte Gentransfertechnik (Lipofektion) in die Verpackungszellen TelCeB6 eingebracht. Die DNA wurde in einem Gesamtvolumen von 100µl in Medium (DMEM) aufgenommen. 2,5µl des Lipofektamins (Fa. Gibco, Eggenstein) wurden ebenfalls in einem Gesamtvolumen von 100µl Medium aufgenommen. Beide Lösungen wurden gemischt und für 30min. bei Raumtemparatur inkubiert, so daß sich Liposomen-DNA-Komplexe bilden konnten. Anschließend wurde mit 800µl Medium aufgefüllt und die Lösung wurde auf die zu transfizierenden Zellen gegeben. Die Zellen wurden für vier Stunden bei 37°C und 5%CO₂ im Brutschrank inkubiert und anschließend erfolgte ein Mediumwechsel (DMEM, 10%FKS, NSP) Die transfizierten Zellen wurden 3 weitere Tage bei 37°C und 5% CO₂ inkubiert, um die Expression des Wildtyp-env-Gens zu ermöglichen. Hierbei erfolgte kein Mediumwechsel.

### 3. Transduktion von Zielzellen mittels verschiedener MLV/SNV-Vektoren

Der Zellüberstand der transfizierten Zellen wurde drei Tage nach der Transfektion in üblicher Weise geerntet und filtriert (0,45µm-Filter), so daß alle Verpackungszellen entfernt wurden. Zwei ml dieses zellfreien Zellüberstand wurde zur Transduktion von 2x10⁵ Zellen der Hunde-Osteosarkom-Zellinie D17 (Watanabe und Temin, *Mol*. *Cell Biol*. 3 (1983), 2241-2249;) in üblicher Weise eingesetzt. Diese Zellen sind permissiv für SNV, wobei der von SNV angesteuerte natürliche Rezeptor bisher unbekannt ist. Die Transduktion wurde in Gegenwart von 40µg/ml Polybren für 4 Stunden durchgeführt. Anschließend wurden die Zellen zwei mal mit 3 ml PBS gewaschen und es erfolgte ein Mediumwechsel. Zur Überprüfung einer erfolgreichen Transduktion wurde nach 72 Stunden ein X-Gal-Test nach der Methode von Sanes et al. (1986) durchgeführt: Der Zellkulturüberstand wurde abgezogen und die Zellen mit PBS ohne (Ca²⁺ und Mg²⁺) gewaschen. Anschließend wurden die Zellen mit einer Fixierlösung (2 % Formaldehyd, 0,2 % Glutaraldehyd in PBS) für 5 min überschichtet und mit PBS gewaschen. Danach wurden die Zellen in 3 ml X-Gal-Reaktionsmixlösung (1 mg/ml, 5 mM K-Ferricyanid, 5 mM K-Ferrocyanid, 2mM MgCl₂) resuspendiert. Nach einer ca. 4-stündigen Inkubation des Ansatzes bei 37° C trat die Blaufärbung der transduzierten Zellen auf Die Blaufärbung läßt auf die Expression der β-Galaktosidase schließen, die nur erfolgen kann, wenn das Expressionskonstrukt MFG-nlsLacZ erfolgreich in die Zielzellen transferiert wurde. Zur Bestimmung des Vektortiters wurden im Mikroskop jeweils 10 Gesichtsfelder der Zellen auf blaue Zellen untersucht. Die durchschnittliche Anzahl der blauen Zellen pro Gesichtsfeld wurde auf die gesamte Fläche des Zellkulturgefäßes (6-Loch-Platte der Fa. Nunc, Wiesbaden, 962mm²) extrapoliert und auf einen ml Zellkulturüberstand standartisiert.) Der in diesem transienten Test erreichte Titer lag bei 2x10⁴ Vektorpartikeln / ml Zellüberstand

### 4. Transduktion von Zielzellen mittels verschiedener MLV/SNV-scFv-Vektoren

Ob ein gezielter Gentransfer mittels verschiedener MLV/SNV-Vektoren mit Antikörpererkennungsfragmenten möglich ist, wurde durch die Kotransfektion von jeweils 2µg DNA SNV-scFv-Expressionskonstrukte und 2µg DNA des für das Wildtyp-ENV-Protein kodierenden Plasmids pIM29 in die Verpackungszellen TelCeB6 getestet. Die verwendeten Expressionskonstrukte pTC53-7A5 zeo, pTC53-M8 zeo, pTC53-K6-2 zeo und pTC53-7E10 zeo enthielten jeweils scFv's, die gegen bisher undefinierte Rezeptoren der lymphoiden Zellinie C8166 (Clapham et al., *Virology* 158 (1987), 44-51) gerichtet sind. Die genannten Konstrukte sind SNV-scFv-ENV Expressionsplasmide der in Beispiel 1 erhaltenen scFv. Die CD4-positiven Zellinien C8166 und Molt4/8 wurde mit dem in Beispiel 3. beschriebenen Verfahren mit zellfreien Überständen transduziert. Durch Transduktion des Pseudotyps [MLV/ SNV pTC53-7E10 zeo] konnte auf C8166-Zellen ein Titer von 5x10³ Vektorpartikel / ml Zellkulturüberstand erreicht werden, während auf HeLa-Zellen (Epithelzellinie) kein Gentransfer im X-Gal-Test nachgewiesen wurde. Somit fand eine zellspezifische Transduktion statt. Bei der Transduktion der vorstehend aufgeführten vier MLV-SNV-scFv-Pseudotypen in Molt4/8 Zellen konnte ebenfalls ein Titer von von 5x10³ Vektorpartikel / ml Zellkulturüberstand erreicht werden.

### 5. Etablierung von stabilen Verpackungszellinien

Zunächst wurde eine MLV-abgeleitete Verpackungszellinie hergestellt, die das wt-SNV-ENV-Protein stabil exprimiert. Das Expressionskonstrukt pIM29 (2µg DNA) und das Hygromycin exprimierende Konstrukt pREP4 (0,1µg DNA) (Invitrogen, Belgien) werden hierfür in die Verpackungszellinie TelCeB6 kotransfiziert. Die experimentelle Vorgehensweise ist unter Punkt 2. beschrieben. Drei Tage nach Transfektion werden die transfizierten Zellen in unterschiedlichen Konzentrationen (1/10, 3/10 und 5/10 des Gesamtvolumens) auf drei 100mm Kulturplatten ausgesät. Die Zellen werden 24 Stunden inkubiert und anschließend wurde ein Mediumwechsel vorgenommen (DMEM, 10% FKS, 200µg/ml Hygromycin). Nicht hygromycin-resistente Zellen lösten sich von dem Zellkulturgefäß ab, so daß einzelne resistente Zell-Kolonien abgenommen werden konnten. Diese Zellklone wurden expandiert und anschließend in einer Transduktion von D17-Zielzellen auf das Vorhandensein des wt-SNV-ENV-Proteins getestet. Die so etablierte TelCeB6-pIM29-hygro Zellinie kann zur Transfektion eines weiteren Expressionskonstruktes benutzt werden. Die Transfektion des chimären SNV-scFv-ENV verläuft analog dem o. g. Verfahren. Zur Selektion eines das chimäre SNV-scFv-ENV stabil exprimierenden Zellklons wird jedoch G418-haltiges DMEM-Medium verwandt, da die chimären env-Konstrukte das Neomycin-Restistengen enthalten.

## Patentansprüche

1. Retroviraler Vektor, enthaltend einen Viruskern ausgewählt aus der Gruppe murines Leukärnievirus (MLV) und eine Virushülle vom Milz-Nekrosevirus (SNV).

2. Vektor nach Anspruch I, wobei die Virushülle das Vollängen-Oberflächenprotein (SU-Protein) des SNV und/oder ein chimäres SNV-virusfremdes Polypeptid-ENV, SNV-HIV-ENV oder SNV-SIV-ENV umfaßt.

3. Vektor nach Anspruch 1 oder 2, wobei das virusfremde Polypeptid einen Ligand, ein Peptidfragment eines Liganden, einen Antikörper, ein Peptidfragment eines Antikörpers oder eine Antikörpererkennungsdomäne (scFv) umfaßt.

4. Vektor nach einem der Ansprüche 1 bis 3, weiter umfassend eine RNA, die in die durch den retroviralen Vektor zu transduzierende Zelle eingeführt werden soll.

5. Vektor nach Anspruch 4, wobei die RNA ein therapeutisches Gen-Transkript oder dessen Nukleinsäurefragment und/oder ein Reportergen und/oder ein Resistenzgen-Transkript umfaßt.

6. Vektor nach Anspruch 5, wobei das therapeutische Gen-Transkript oder dessen Nukleinsäurefragment das CFTR-, phox91-, ADA-, IL-16-, p53- oder revM10-Gen-Transkript und ein oder mehrere Impfgen-Transkripte z. B. rekombinantes gp120 und IL-16 umfaßt.

7. Vektor nach Anspruch 5, wobei das Reportergen-Transkript ,β-Galaktosidase, "Green Flourescent Protein", Luciferase und das Resistenzgen-Transkript Neomycin oder "multiple drug resistence gene" umfaßt.

8. Vektor nach einem der Ansprüche 1 bis 7 zur Verwendung als Arzneimittel.

9. Verwendung des Vektors nach einem der Ansprüche 1-7 zur Herstellung eines Arzneimittels zur somatischen Gentherapie, Impftherapie oder Diagnostik.

10. Verwendung des Vektors nach Anspruch 8 zur Herstellung eines Arzneimittels zur Therapie der Cystischen Fibrose, des ADA-Mangels, der chronischen Granulomatose oder der HIV-1 Infektion.

11. Retrovirale Verpackungszelle zur Gewinnung der retroviralen Vektoren nach einem der Ansprüche 1 bis 7, transformiert sowohl mit einem oder mehreren psi-negativen Expressionskonstrukt(en), die die gag- und pol-Genprodukte des MLV. exprimieren, als auch mit einem psi-negativen SNV-Env-Expressionskonstrukt und/oder psi-negativen SNV-Env-virusfremden Polypeptid-, SNV-HIV-ENV- oder SNV-SIV-ENV-Expressionskonstrukt.

12. Verpackungszelle nach Anspruch 11, wobei das virusfremde Polypeptid einen Liganden, ein Peptidfragment eines Liganden, einen Antikörper, ein Peptidfragment eines Antikörpers oder eine Antikörpererkennungsdomäne (scFv) umfaßt.

13. Verpackungszelle nach Anspruch 11, ferner umfassend ein psi-positives Expressionskonstrukt, umfassend eine Nukleinsäuresequenz, die in die durch den retroviralen Vektor zu transduzierende Zelle eingeführt werden soll.

14. Verpackungszelle nach Anspruch 13, wobei die Nukleinsäuresequenz ein therapeutisches Gen oder dessen Nukleinsäurefragment und/oder ein Reportergen und/oder ein Resistenzgen umfaßt.

15. Verpackungszelle nach Anspruch 14, wobei das therapeutische Gen oder dessen Nukleinsäurefragment das CFTR-, phox91-, ADA-, IL-16-, p53- oder revM10-Gen oder ein oder mehrere Impfgene z. B. rekombinantes gp120 und IL-16 umfaßt.

16. Verpackungszelle nach Anspruch 14, wobei das Reportergen ausgewählt ist aus der Gruppe β-Galaktosidase, "Green Fluorescent Protein", Luciferase und das Resistenzgen aus der Gruppe Neomycin oder "multiple drug resistence gene".

## Claims

1. Retroviral vector, containing a virus core selected from the group consisting of murine leukemia virus (MLV) and a virus envelope of spleen nekrosis virus (SNV).

2. The vector of claim 1, wherein said virus envelope comprises the full-length surface protein (SU protein) of SNV and/or a chimeric SNV virus external polypeptide-ENV, SNV-HIV-ENV or SNV-SIV-ENV.

3. The vector of claim 1 or 2, wherein the virus-external polypeptide comprises a ligand, a peptide fragment of a ligand, an antibody, a peptide fragment of an antibody or an antibody recognition domain (scFv).

4. The vector of any one of claims 1 to 3, further comprising a RNA which shall be introduced into the cell to be transduced with said retroviral vector.

5. The vector of claim 4, wherein said RNA comprises a therapeutic gene transcript or a nucleic acid fragment thereof and/or a reporter gene and/or a resistance gene transcript.

6. The vector of claim 5, wherein said therapeutic gene transcript or a nucleic acid fragment thereof comprises the CFTR, phox91, ADA, IL-16, p53 or revM10 gene transcript and one or more vaccine gene transcripts, e.g. recombinant gp120 and IL-16.

7. The vector of claim 5, wherein said reporter gene transcript comprises β-galactosidase, green fluorescent protein, luciferase and the resistance gene transcript neomycin or multiple drug resistance gene.

8. The vector according to any one of claims 1 to 7 for use as medicament.

9. Use of a vector according to any one of claims 1 to 7 for the preparation of a medicament for somatic gene therapy, vaccination therapy or diagnostics.

10. Use of the vector according to claim 8 for the preparation of a medicament for therapy of cystic fibrosis, ADA deficiency, chronic granulomatosis or HIV-1 infection.

11. Retroviral packaging cell for production of the retroviral vectors according to any one of claims 1 to 7, transformed with both one or more psi-negative expression construct(s) expressing the gag and pol gene products of MLV and a psi-negative SNV-Env expression construct and/or a psi-negative SNA Env virus external polypeptide, SNV-HIV-ENV or SNV-SIV-ENV expression construct.

12. The retroviral packaging cell of claim 11, wherein said virus external polypeptide comprises a ligand, a peptide fragment of a ligand, an antibody, a peptide fragment of an antibody or an antibody recognition domain (scFv).

13. The retroviral packaging cell of claim 11, further comprising a psi-positive expression construct comprising a nucleic acid sequence which shall be introduced into the cell to be transduced with said retroviral vector.

14. The retroviral packaging cell of claim 13, wherein said nucleic acid sequence comprises a therapeutic gene or a nucleic acid fragment thereof and/or a reporter gene and/or a resistance gene.

15. The retroviral packaging cell of claim 14, wherein said therapeutic gene transcript or a nucleic acid fragment thereof comprises the CFTR, phox91, ADA, IL-16, p53 or revM10 gene transcript and one or more vaccine gene transcripts, e.g. recombinant gp120 and IL-16.

16. The retroviral packaging cell of claim 14, wherein said reporter gene transcript is selected from the group consisting of β-galactosidase, green fluorescent protein, luciferase and the resistance gene transcript from the group of neomycin or multiple drug resistance gene.

## Revendications

1. Vecteur rétroviral, contenant un noyau viral choisi dans le groupe du virus de la leucémie murine (MLV) et une enveloppe virale du virus de la nécrose splénique (SNV).

2. Vecteur selon la revendication 1, dans lequel l'enveloppe virale comprend la protéine de surface entière (protéine SU) du SNV et/ou un polypeptide-ENV, SNV-HIV-ENV ou SNV-SIV-ENV chimérique étranger au virus SNV.

3. Vecteur selon la revendication 1 ou 2, dans lequel le polypeptide étranger au virus comprend un ligand, un fragment peptidique d'un ligand, un anticorps, un fragment peptidique d'un anticorps ou un domaine de reconnaissance d'anticorps (scFv).

4. Vecteur selon l'une des revendications 1 à 3, comprenant en plus un ARN destiné à être introduit dans la cellule à transfecter par le vecteur rétroviral.

5. Vecteur selon la revendication 4, dans lequel l'ARN comprend un produit de transcription de gène thérapeutique ou son fragment d'acides nucléiques et/ou un gène reporter et/ou un produit de transcription de gène de résistance.

6. Vecteur selon la revendication 5,. dans lequel le produit de transcription de gène thérapeutique ou son fragment d'acides nucléiques comprend le produit de transcription de gène CFTR, phox91, ADA, IL-16-, p53 ou revM10 et un ou plusieurs produits de transcription de gènes vaccinaux, par exemple de la gp120 recombinante et de l'IL-16.

7. Vecteur selon la revendication 5, dans lequel le produit de transcription de gène reporter comprend la β-galactosidase, la "*green fluorescent protein",* la luciférase et le produit de transcription de gène de résistance néomycine ou le "*multiple drug resistance gene*".

8. Vecteur selon l'une des revendications 1 à 7, pour son utilisation comme médicament.

9. Utilisation du vecteur selon l'une des revendications 1 à 7 pour la préparation d'un médicament destiné à la thérapie génique somatique, à la thérapie vaccinale ou au diagnostic.

10. Utilisation du vecteur selon la revendication 8 pour la préparation d'un médicament destiné à la thérapie de la mucoviscidose, du déficit en ADA, de la granulomatose chronique ou de l'infection par le HIV-1.

11. Cellule d'encapsidation rétrovirale pour l'obtention des vecteurs rétroviraux selon l'une des revendications 1 à 7, transformée à la fois avec un ou plusieurs produits d'assemblage d'expression psi-négatifs qui expriment les produits géniques gag et pol du MLV et avec un produit d'assemblage d'expression de SNV-Env psi-négatif et/ou un produit d'assemblage d'expression de polypeptide, SNV-HIV-ENV ou SNV-SIV-ENV psi-négatif étranger au virus SNV-Env.

12. Cellule d'encapsidation selon la revendication 11, dans laquelle le polypeptide étranger au virus comprend un ligand, un fragment peptidique d'un ligand, un anticorps, un fragment peptidique d'un anticorps ou un domaine de reconnaissance d'anticorps (scFv).

13. Cellule d'encapsidation selon la revendication 11, comprenant en plus un produit d'assemblage d'expression psi-positif comprenant une séquence d'acides nucléiques destiné à être introduite dans la cellule à transfecter par le vecteur rétroviral.

14. Cellule d'encapsidation selon la revendication 13, dans laquelle la séquence d'acides nucléiques comprend un gène thérapeutique ou son fragment d'acides nucléiques et/ou un gène reporter et/ou un gène de résistance.

15. Cellule d'encapsidation selon la revendication 14, dans laquelle le gène thérapeutique ou son fragment d'acides nucléiques comprend le gène CFTR, phox91, ADA, IL-16-, p53 ou revM10 et un ou plusieurs gènes vaccinaux, par exemple de la gp120 recombinante et de l'IL-16.

16. Cellule d'encapsidation selon la revendication 14, dans laquelle le gène reporter est choisi dans le groupe comprenant la β-galactosidase, la "*green fluorescent protein",* la luciférase, et le gène de résistance est choisi dans le groupe comprenant la néomycine ou le "*multiple drug resistance gene*".
